# FASCICULE DE BREVET EUROPEEN

(11) **EP 1 092 424 B1**
(45) Date de publication et mention de la délivrance du brevet: **13.03.2002**
(21) Numéro de dépôt: 00402324.8
(22) Date de dépôt: 21.08.2000
(51) Int. Cl.: A61K 7/48, A61K 7/02, A61K 7/021, A61K 9/107, A61K 7/00

(54) **Composition sous forme d'émulsion eau-dans-huile, contenant des fibres, et son utilisation dans le domaine cosmétique**
W/O-Emulsion enthaltend Fibern und ihre Verwendung in der Kosmetik
W/O-Emulsion containig fibres and its use in cosmetics

(30) Priorité: 15.10.1999 FR 9912910
(43) Date de publication de la demande: 18.04.2001
(73) Titulaire: L'OREAL, 75008 Paris (FR)
(72) Inventeur: Afriat, Isabelle, 75003 Paris (FR)
(74) Mandataire: Rasson, Catherine

(56) Documents cités:
- EP-A- 0 336 900
- WO-A-98/50005
- US-A- 4 659 562

## Description

L'invention se rapporte à une composition se présentant sous forme d'une émulsion eau-dans-huile (E/H) contenant des fibres, un tensioactif siliconé et une argile, et à l'utilisation cosmétique de la dite composition, en particulier pour le soin, le traitement et/ou le maquillage de la peau du corps ou du visage, des cheveux, des cils et/ou des lèvres.

Il est connu par le document JP07-196440 des compositions cosmétiques contenant des fibres de polyamide courtes, celles-ci donnant aux dites compositions un toucher velouté et une bonne tenue cosmétique. Toutefois, les emulsions E/H décrites dans ce document, et par exemple dans les exemples 4 et 5, ont une stabilité limitée dans le temps et insuffisante pour un produit cosmétique. En outre, celle de l'exemple 4 présente un toucher gras.

Il subsiste donc le besoin d'émulsions E/H contenant des fibres, qui soient stables tout en présentant de bonnes propriétés cosmétiques et donc qui n'aient pas les inconvénients de celles de l'art antérieur.

La demanderesse a découvert de façon inattendue que l'association d'un tensioactif siliconé et d'argile permettait de réaliser des émulsions eau-dans-huile contenant des fibres, stables et cosmétiquement agréables, à savoir douces et non grasses. En outre, on peut incorporer dans les compositions selon l'invention, une quantité de fibres plus importante que décrit dans le document JP07-196440.

L'invention a pour objet une composition sous forme d'émulsion comprenant, dans un milieu physiologiquement acceptable, une phase aqueuse dispersée dans une phase huileuse, caractérisée en ce qu'elle contient des fibres, au moins un tensioactif siliconé et au moins une argile.

On entend par « milieu physiologiquement acceptable » un milieu compatible avec la peau, les lèvres, les ongles, le cuir chevelu et/ou les cheveux.

La composition obtenue selon l'invention présente une bonne stabilité dans le temps, même à une température supérieure à la température ambiante (par exemple 45°C). Cette composition a l'aspect d'une crème (produit souple par opposition à un produit solide) et elle a une texture veloutée, agréable à l'application.

Les fibres utilisables dans la composition de l'invention peuvent être des fibres d'origine synthétique ou naturelle, minérale ou organique. Elles peuvent être courtes ou longues, unitaires ou organisées par exemple tressées. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 nm à 20 mm, de préférence de 10 nm à 5 mm et mieux de 0,1 mm à 1,5 mm. Leur section peut être comprise dans un cercle de diamètre allant de 2 nm à 100 µm, de préférence allant de 20 nm à 20 µm et mieux de 5 µm à 50 µm. Le poids des fibres est souvent donné en denier ou décitex.

Les fibres peuvent être celles utilisées dans la fabrication des textiles et notamment des fibres de soie, de coton, de laine, de lin, de cellulose extraites notamment du bois, des légumes ou des algues, de polyamide (Nylon® ), de rayonne, de viscose, d'acétate notamment d'acétate de rayonne, d'acétate de cellulose ou d'acétate de soie, de poly-p-phénylène téréphtamide notamment de Kevlar® , en acrylique notamment de polyméthacrylate de méthyle ou de poly-2-hydroxyéthylméthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester, et les mélanges de ces fibres.

On peut aussi utiliser les fibres chirurgicales comme les fibres synthétiques résorbables préparées à partir d'acide glycolique et de caprolactone (« Monocryl » de la société Johnson & Johnson) ; les fibres synthétiques résorbables du type copolymère d'acide lactique et d'acide glycolique (« Vicryl » de la société Johnson & Johnson) ; les fibres de polyester téréphtalique (« Ethibond » de la société Johnson & Johnson) et les fils d'acier inoxydable (« Acier » de la société Johnson & Johnson).

Par ailleurs, les fibres peuvent être traités ou non en surface, enrobées ou non. Comme fibres enrobées utilisables dans l'invention, on peut citer des fibres de polyamide enrobées de sulfure de cuivre pour un effet antistatique (par exemple les fibres R-STAT de la société Rhodia) ou un autre polymère permettant une organisation particulière des fibres (traitement de surface spécifique) ou traitement de surface induisant des effets de couleurs/hologrammes (fibre « Lurex » de la société Sildorex, par exemple).

Les fibres utilisables dans la composition selon l'invention sont de préférence des fibres de polyamide ou de poly-p-phénylène téréphtamide. Leur longueur peut aller de 0,1 à 5 mm, de préférence de 0,25 à 1,6 mm, et leur diamètre moyen peut aller de 5 à 50 µm. En particulier, on peut utiliser les fibres de polyamide commercialisées par les Etablissements P. Bonte sous le nom Polyamide 0.9 Dtex 0.3 mm, ayant un diamètre moyen de 6 µm, un poids d'environ 0.9 dtex et une longueur allant de 0,3 mm à 1,5 mm. On peut aussi utiliser les fibres de poly-p-phénylène téréphtamide de diamètre moyen de 12 µm et de longueur d'environ 1,5 mm comme celles vendues sous le nom de Kevlar Floc par la société Du Pont Fibres.

Les fibres peuvent être présentes dans la composition selon l'invention en une quantité allant de 0,1 à 20 % en poids et de préférence de 0,5 à 12 % en poids par rapport au poids total de la composition.

Comme tensioactifs siliconés pouvant entrer dans la composition selon l'invention, on peut citer les dimethicone copolyols et les alkyldiméthicone copolyols. Comme dimethicone copolyol, on peut citer par exemple le mélange de dimethicone copolyol, de cyclomethicone et d'eau (10/88/2), commercialisé par la société Dow Corning sous la dénomination DC3225C ou DC2-5225C, et le mélange de dimethicone copolyol et de cyclopentasiloxane (85/15) commercialisé sous la dénomination Abil EM-97 par la société Goldschmidt. Comme alkyldiméthicone copolyol, on peut citer notamment ceux ayant un radical alkyle comportant de 10 à 22 atomes de carbone, tel que le cétyl diméthicone copolyol comme le produit commercialisé sous la dénomination Abil EM-90 par la société Goldschmidt ; le lauryl diméthicone copolyol et par exemple le mélange d'environ 91 % de lauryl diméthicone copolyol et d'environ 9 % d'alcool isostéarylique, commercialisé sous la dénomination Q2-5200 par la société Dow Corning, et les mélanges de ces tensioactifs siliconés. Le tensioactif siliconé est de préférence un diméthicone copoyol.

La quantité de tensioactif(s) siliconé(s) dans la composition de l'invention va de préférence de 0,1 à 5 % en poids de matière active, et mieux de 0,5 à 2 % en poids de matière active par rapport au poids total de la composition.

A titre d'exemples d'argiles utilisables dans la composition de l'invention, on peut citer les argiles de la famille de la kaolinite telles que la kaolinite, la dickite, la nacrite, les argiles de la famille de l'halloysite, de la dombassite, de l'antigorite, de la benthiérine, de la pyrophyllite, des montmorillonites, de la beidellite, des vermiculites, du talc, de la stévensite, des hectorites éventuellement modifiées (smectite), des saponites, des chlorites, de la sépiolite.

Les argiles peuvent également être modifiées chimiquement par divers composés tels que les acides acryliques, les polysaccharides (par exemple la carboxyméthylcellulose) ou les cations organiques.

Selon un mode particulièrement préféré de réalisation de la présente invention, l'argile mise en oeuvre est choisie parmi la kaolinite, les montmorillonites, les hectorites et leurs mélanges. On utilise encore plus particulièrement une hectorite modifiée, et par exemple une bentone comme le mélange « cyclomethicone, Quaternium-18 hectorite, SD alcohol 40 » (10/85/5) (nom CTFA) commercialisé sous la dénomination Bentone Gel VS-5 par la société Rheox.

La quantité d'argile(s) dans la composition de l'invention va généralement de 0,1 à 10 % en poids, et particulièrement de 0,2 à 5 % en poids, encore plus préférentiellement de 0,3 à 1 % en poids par rapport au poids total de la composition.

La phase huileuse de la composition selon l'invention peut renfermer toute sorte d'huiles et de corps gras bien connus de l'homme du métier, comme exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkylbenzoates), les huiles de silicone volatiles ou non volatiles, et les huiles fluorées ou fluorosiliconées, ainsi que les mélanges de ces huiles.

De préférence, la phase huileuse de la composition de l'invention comprend au moins une huile de silicone qui peut être présente en une quantité allant par exemple de 5 à 50 % en poids et de préférence de 9 à 30 % en poids par rapport au poids total de la composition. Comme huile de silicone, on peut citer par exemple les huiles de silicone volatiles telles que les cyclodiméthylsiloxanes ou cyclométhicones, comme la pentacyclométhicone, la tétracyclométhicone ou l'hexacyclométhicone; les huiles de silicone non volatiles telles que les polydiméthylsiloxanes (PDMS). De préférence, la composition de l'invention contient au moins une huile de silicone volatile.

La phase huileuse peut contenir, en outre, d'autres constituants gras tels que les alcools gras comme l'alcool stéarylique, l'alcool cétylique et l'alcool cétéarylique, les acides gras, les gommes, par exemple les gommes de silicone comme le mélange PDMS à groupements alpha oméga hydroxylés / PDMS 5 cst (12/88) vendu sous la dénomination DC 1503 par la société Dow Coming, et les gélifiants lipophiles tels que la bentone.

La phase huileuse est présente dans la composition selon l'invention en une quantité allant généralement de 10 à 50 % et de préférence de 12 à 40 % en poids par rapport au poids total de la composition, cette quantité comprenant la quantité de tensioactif siliconé.

La phase aqueuse de la composition de l'invention peut aller de 30 à 85 % en poids et de préférence de 40 à 75 % en poids par rapport au poids total de la composition, et peut contenir, outre l'eau, des solvants tels que les alcools primaires comportant de 1 à 4 atomes de carbone comme l'éthanol, ou les polyols comme le butylène glycol. Le ou les solvants peuvent être présents en une quantité allant de 0,1 à 30 % en poids par rapport au poids total de la composition.

La composition de l'invention peut contenir aussi des gélifiants lipophiles tels que les organopolysiloxanes élastomères comme par exemple ceux commercialisés sous les noms KSG 6 de Shin-Etsu, Trefil E-505C ou Trefil E-506C de Dow-Corning, Gransil (SR-CYC, SR DMF10, SR-DC556) de Grant Industries, ou ceux commercialisés sous forme de gels déjà constitués : KSG 15, KSG 17, KSG 16, KSG 18 de Shin-Etsu, Gransil SR 5CYC gel, Gransil SR DMF 10 gel, Gransil SR DC556 gel de Grant Industries, 1229-02-167 et 1229-02-168 de General Electric. On peut aussi utiliser un mélange de ces produits commerciaux.

La composition selon l'invention se présente sous forme d'une crème et peut constituer notamment une compositon cosmétique ou dermatologique. Elle trouve alors son application dans un grand nombre de traitements notamment cosmétiques de la peau, y compris du cuir chevelu, des cheveux, des ongles, et/ou des muqueuses, en particulier pour le soin, le nettoyage, le maquillage et/ou la protection solaire de la peau et/ou des muqueuses.

Aussi, la présente invention a pour objet l'utilisation cosmétique de la composition telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition telle que définie ci-dessus.

De façon connue, la composition de l'invention peut contenir également des adjuvants habituels dans les domaines cosmétique et/ou dermatologique, tels que les actifs, les conservateurs, les antioxydants, les agents complexants, les ajusteurs de pH (acides ou basiques), les parfums, les charges, les bactéricides, les absorbeurs d'odeur, les matières colorantes (pigments et colorants) et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme actifs, on peut citer notamment les hydratants et par exemple les hydrolysats de protéines et les polyols tels que la glycérine, les glycols comme les polyéthylène glycols, et les dérivés de sucre.

Le ou les actifs peuvent être par exemple présents en une concentration allant de 0,01 à 20 %, de préférence de 0,1 à 5 % et mieux de 0,5 à 3 % du poids total de la composition.

Les exemples ci-après de compositions selon l'invention sont donnés à titre d'illustration. Les quantités y sont données en % en poids, sauf mention contraire.

### Exemple 1 : crème protectrice

### A. Phase huileuse

- Diméthicone copolyol
   (DC2-5225C vendu par la société Dow Corning) 10 %
- Huile de silicone volatile (cyclopentadiméthylsiloxane) 6 %
- Bentone (smectite : Bentone GEL VS-5V de la société
   RHEOX) 1 %
- Huile fluorosiliconée (fluoro-propyl dimethylsiloxane)
   (X-22-819 vendu par la société Shin Etsu) 4 %

### B. Phase aqueuse

- Ethanol 5 %
- Sulfate de magnésium 0,7 %
- Glycérine 10 %
- Conservateur 1 %
- Eau qsp 100 %
Fibres de polyamide
(Polyamide 0,9 Dtex, 0,3 mm - Société Paul Bonte) 5 %

Mode opératoire : On introduit à froid les fibres dans la phase huileuse et on fait l'émulsion en versant la phase aqueuse dans la phase huileuse sous forte agitation.

On obtient une crème blanche apte à protéger la peau.

### Exemple 2 : crème hydratante

### A. Phase huileuse

- Diméthicone copolyol (DC2-5225C vendu
   par la société Dow Corning) 10 %
- Huile de silicone volatile (cyclopentadiméthylsiloxane) 6 %
- Bentone (smectite : Bentone GEL VS-5V de la société
   RHEOX) 2,5 %
- Gomme de silicone (DC 1503 vendu par la société Dow Corning) 2,5 %
- Huile fluorosiliconée (fluoro-propyl dimethylsiloxane)
   (X-22-819 vendu par la société Shin Etsu) 4 %

### B. Phase aqueuse

- Ethanol 5 %
- Sulfate de magnésium 0,7 %
- Glycérine 10 %
- Conservateur 1 %
- Eau qsp 100 %
Fibres de polyamide
(Polyamide 0,9 Dtex, 0,3 mm - Société Paul Bonte) 5 %

Mode opératoire : On introduit à froid les fibres dans la phase huileuse et on fait l'émulsion en versant la phase aqueuse dans la phase huileuse sous forte agitation.

On obtient une crème blanche apte à hydrater la peau.

### Exemple 3 : fond de teint

### A. Phase huileuse

- Diméthicone copolyol (DC2-5225C vendu
   par la société Dow Corning) 10 %
- Huile de silicone volatile (cyclopentadiméthylsiloxane) 6 %
- Dimethicone/vinyl dimethicone crosspolymer
   and dimethicone (KSG 16 de la société Shin-Etsu) 4 %
- Bentone (smectite : Bentone GEL VS-5V de la société
   RHEOX) 2 %
- Huile fluorosiliconée (fluoro-propyl dimethylsiloxane)
   (X-22-819 vendu par la société Shin Etsu) 4 %
- Colorants 0,02 %

### B. Phase aqueuse

- Ethanol 5 %
- Sulfate de magnésium 0,7 %
- Sodium EDTA 0,1 %
- Glycérine 5 %
- Conservateur 1 %
- Eau qsp 100 %

### C. Phase C

- Fibres de polyamide
   (Polyamide 0,9 Dtex, 0,3 mm - Société Paul Bonte) 15 %
- Dioxyde de titane 0,9 %
- Oxydes de fer 0,2 %

Mode opératoire : On introduit à froid la phase C dans la phase huileuse que l'on broie, et on fait l'émulsion en versant la phase aqueuse dans la phase huileuse sous forte agitation.

On obtient une crème apte à donner bonne mine.

## Revendications

1. Composition sous forme d'émulsion comprenant, dans un milieu physiologiquement acceptable, une phase aqueuse dispersée dans une phase huileuse, **caractérisée en ce qu'**elle contient des fibres, au moins un tensioactif siliconé et au moins une argile.

2. Composition selon la revendication précédente, **caractérisée en ce que** les fibres sont choisies parmi les fibres de soie, de coton, de laine, de lin, de cellulose extraites notamment du bois, des légumes ou des algues, de polyamide, de rayonne, de viscose, d'acétate notamment d'acétate de rayonne, d'acétate de cellulose ou d'acétate de soie, de poly-p-phénylène téréphtamide, en acrylique notamment de polyméthacrylate de méthyle ou de poly-2-hydroxyéthylméthacrylate, de polyoléfine et notamment de polyéthylène ou de polypropylène, de verre, de silice, d'aramide, de carbone notamment sous forme graphite, de Téflon ® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, des fibres chirurgicales, et leurs mélanges.

3. Composition selon l'une quelconque des revendications précédentes, **caractérisée en ce que** les fibres sont des fibres d'origine synthétique.

4. Composition selon l'une quelconque des revendications précédentes, **caractérisée en ce que** les fibres sont des fibres de polyamide ou de poly-p-phénylène téréphtamide.

5. Composition selon l'une quelconque des revendications précédentes, **caractérisée en ce que** les fibres ont une longueur allant de 0,1 à 1,5 mm.

6. Composition selon l'une quelconque des revendications précédentes, **caractérisée en ce que** les fibres ont un diamètre moyen allant de 5 à 50 µm.

7. Composition selon l'une quelconque des revendications précédentes, **caractérisée en ce que** les fibres sont présentes en une quantité allant de 0,1 à 20 % en poids par rapport au poids total de la composition.

8. Composition selon l'une quelconque des revendications précédentes, **caractérisée en ce que** le tensioactif siliconé est choisi parmi les dimethicone copolyols.

9. Composition selon l'une quelconque des revendications précédentes, **caractérisée en ce que** la quantité de tensioactif siliconé va de 0,1 à 5 % en poids de matière active par rapport au poids total de la composition.

10. Composition selon l'une quelconque des revendications précédentes, **caractérisée par le fait que** l'argile est choisie parmi la kaolinite, la dickite, la nacrite, les argiles de la famille de l'halloysite, de la dombassite, de l'antigorite, de la benthiérine, de la pyrophyllite, des montmorillonites, de la beidellite, des vermiculites, du talc, de la stévensite, des hectorites éventuellement modifiées (smectite), des saponites, des chlorites, de la sépiolite, et leurs mélanges.

11. Composition selon l'une quelconque des revendications précédentes, **caractérisée par le fait que** l'argile est une bentone.

12. Composition selon l'une quelconque des revendications précédentes, **caractérisée par le fait que** la quantité d'argile va de 0,1 à 10 % en poids par rapport au poids total de la composition.

13. Composition selon l'une quelconque des revendications précédentes, **caractérisée par le fait que** la phase huileuse est présente en une quantité allant de 10 à 50 % en poids par rapport au poids total de la composition.

14. Composition selon l'une quelconque des revendications précédentes, **caractérisée par le fait que** la phase huileuse contient au moins une huile de silicone.

15. Composition selon l'une quelconque des revendications précédentes, **caractérisée par le fait qu'**elle constitue une composition cosmétique ou dermatologique.

16. Utilisation cosmétique de la composition selon l'une quelconque des revendications 1 à 15, pour le traitement, la protection, le soin, le démaquillage et/ou le nettoyage de la peau, des lèvres et/ou des cheveux, et/ou pour le maquillage de la peau et/ou des lèvres.

17. Procédé de traitement cosmétique de la peau, y compris du cuir chevelu, des cheveux, et/ou des lèvres, **caractérisé par le fait que** l'on applique sur la peau, les cheveux et/ou les lèvres, une composition selon l'une quelconque des revendications à 15.

## Claims

1. Composition in emulsion form comprising, in a physiologically acceptable medium, an aqueous phase dispersed in an oily phase, **characterized in that** it contains fibres, at least one silicone surfactant and at least one clay.

2. Composition according to the preceding claim, **characterized in that** the fibres are chosen from silk, cotton, wool or flax fibres, cellulose fibres extracted in particular from wood, plants or algae, polyamide, rayon or viscose fibres, acetate fibres, in particular rayon acetate, cellulose acetate or silk acetate fibres, poly-p-phenylene terephthamide fibres, acrylic fibres, in particular polymethyl methacrylate or poly-2-hydroxyethyl methacrylate fibres, polyolefin fibres and in particular polyethylene or polypropylene fibres, glass, silica or aramid fibres, carbon fibres, in particular in graphite form, Teflon® , insoluble collagen, polyester, polyvinyl chloride or polyvinylidene chloride, polyvinyl alcohol, polyacrylonitrile, chitosan, polyurethane or polyethylene phthalate fibres, fibres from mixtures of polymers, surgical fibres, and mixtures thereof.

3. Composition according to either of the preceding claims, **characterized in that** the fibres are fibres of synthetic origin.

4. Composition according to any one of the preceding claims, **characterized in that** the fibres are polyamide fibres or poly-p-phenylene terephthamide fibres.

5. Composition according to any one of the preceding claims, **characterized in that** the fibres have a length ranging from 0.1 to 1.5 mm.

6. Composition according to any one of the preceding claims, **characterized in that** the fibres have an average diameter ranging from 5 to 50 µm.

7. Composition according to any one of the preceding claims, **characterized in that** the fibres are present in an amount ranging from 0.1 to 20% by weight relative to the total weight of the composition.

8. Composition according to any one of the preceding claims, **characterized in that** the silicone surfactant is chosen from dimethicone copolyols.

9. Composition according to any one of the preceding claims, **characterized in that** the amount of silicone surfactant ranges from 0.1 to 5% by weight of active material relative to the total weight of the composition.

10. Composition according to any one of the preceding claims, **characterized in that** the clay is chosen from kaolinite, dickite or nacrite, and clays of the halloysite, dombassite, antigorite, benthierine, pyrophyllite, montmorillonite, beidellite, vermiculite, talc, stevensite, optionally modified hectorite (smectite), saponite, chlorite or sepiolite family, and mixtures thereof.

11. Composition according to any one of the preceding claims, **characterized in that** the clay is a bentone.

12. Composition according to any one of the preceding claims, **characterized in that** the amount of clay ranges from 0.1 to 10% by weight relative to the total weight of the composition.

13. Composition according to any one of the preceding claims, **characterized in that** the oily phase is present in an amount ranging from 10 to 50% by weight relative to the total weight of the composition.

14. Composition according to any one of the preceding claims, **characterized in that** the oily phase contains at least one silicone oil.

15. Composition according to any one of the preceding claims, **characterized in that** it constitutes a cosmetic or dermatological composition.

16. Cosmetic use of the composition according to any one of Claims 1 to 15, for treating, protecting, caring for, removing make-up from and/or cleansing the skin, the lips and/or the hair, and/or for making up the skin and/or the lips.

17. Cosmetic treatment process for the skin, including the scalp, the hair and/or the lips, **characterized in that** a composition according to any one of Claims 1 to 15 is applied to the skin, the hair and/or the lips.

## Patentansprüche

1. Zusammensetzung, die in Form einer Emulsion vorliegt und die in einem physiologisch akzeptablen Medium eine in einer Ölphase dispergierte wäßrige Phase enthält, **dadurch gekennzeichnet, daß** sie Fasern, mindestens ein grenzflächenaktives Silicon und mindestens einen Ton enthält.

2. Zusammensetzung nach dem vorhergehenden Anspruch, **dadurch gekennzeichnet, daß** die Fasern unter den Fasern aus Seide, Baumwolle, Wolle, Leinen, Cellulosefasern, die insbesondere aus Holz, Gemüse oder Algen gewonnen wurden, Fasern aus Polyamid, Reyon, Viscose, Acetat und insbesondere dem Acetat von Reyon, Cellulose oder Seide, Poly-p-phenylenterephthamid, Acrylfasern, insbesondere aus Polymethylmethacrylat oder Poly-2-hydroxyethylmethacrylat, Fasern aus Polyolefin und insbesondere Polyethylen oder Polypropylen, Glas, Siliciumdioxid, Aramid, Kohlenstoff und insbesondere Kohlenstoff in Form von Graphit, Teflon® , unlöslichem Kollagen, Polyestern, Polyvinylchlorid, Polyvinylidenchlorid, Polyvinylalkohol, Polyacrylnitril, Chitosan, Polyurethan und Polyethylenphthalat, Fasern aus Polymergemischen, chirurgischen Fasern und deren Gemischen ausgewählt sind.

3. Zusammensetzung nach einem der vorhergehenden Ansprüche, **dadurch gekennzeichnet, daß** es sich bei den Fasern um Fasern synthetischer Herkunft handelt.

4. Zusammensetzung nach einem der vorhergehenden Ansprüche, **dadurch gekennzeichnet, daß** die Fasern Polyamidfasern oder Fasern aus Poly-p-phenylenterephthamid sind.

5. Zusammensetzung nach einem der vorhergehenden Ansprüche, **dadurch gekennzeichnet, daß** die Fasern eine Länge von 0,1 bis 1,5 mm aufweisen.

6. Zusammensetzung nach einem der vorhergehenden Ansprüche, **dadurch gekennzeichnet, daß** die Fasern einen mittleren Durchmesser von 5 bis 50 µm aufweisen.

7. Zusammensetzung nach einem der vorhergehenden Ansprüche, **dadurch gekennzeichnet, daß** die Fasern in einem Mengenanteil von 0,1 bis 20 Gew.-%, bezogen auf das Gesamtgewicht der Zusammensetzung, vorliegen.

8. Zusammensetzung nach einem der vorhergehenden Ansprüche, **dadurch gekennzeichnet, daß** das grenzflächenaktive Silicon unter den Dimethiconcopolyolen ausgewählt ist.

9. Zusammensetzung nach einem der vorhergehenden Ansprüche, **dadurch gekennzeichnet, daß** das grenzflächenaktive Silicon in einem Mengenanteil von 0,1 bis 5 Gew.-% Wirkstoff, bezogen auf das Gesamtgewicht der Zusammensetzung, vorliegt.

10. Zusammensetzung nach einem der vorhergehenden Ansprüche, **dadurch gekennzeichnet, daß** der Ton unter Kaolinit, Dickit, Nakrit und Tonen aus den folgenden Gruppen: Halloysit, Donbassit, Antigorit, Berthierin, Pyrophyllit, Montmorillonite, Beidellit, Vermiculite, Talk, Stevensit, Hectorite, die gegebenenfalls modifiziert sind (Smektit), Saponite, Chlorite und Sepiolith und deren Gemischen ausgewählt ist.

11. Zusammensetzung nach einem der vorhergehenden Ansprüche, **dadurch gekennzeichnet, daß** es sich bei dem Ton um Bentone handelt.

12. Zusammensetzung nach einem der vorhergehenden Ansprüche, **dadurch gekennzeichnet, daß** der Mengenanteil des Tons im Bereich von 0,1 bis 10 Gew.-%, bezogen auf das Gesamtgewicht der Zusammensetzung, liegt.

13. Zusammensetzung nach einem der vorhergehenden Ansprüche, **dadurch gekennzeichnet, daß** die Ölphase in einer Menge von 10 bis 50 Gew.-%, bezogen auf das Gesamtgewicht der Zusammensetzung, vorliegt.

14. Zusammensetzung nach einem der vorhergehenden Ansprüche, **dadurch gekennzeichnet, daß** die Ölphase mindestens ein Siliconöl enthält.

15. Zusammensetzung nach einem der vorhergehenden Ansprüche, **dadurch gekennzeichnet, daß** es sich um eine kosmetische oder dermatologische Zusammensetzung handelt.

16. Kosmetische Verwendung der Zusammensetzung nach einem der Ansprüche 1 bis 15 zur Behandlung, zum Schutz, zur Pflege, zum Abschminken und/oder zur Reinigung der Haut, der Lippen und/oder der Haare und/oder zum Schminken der Haut und/oder der Lippen.

17. Verfahren zur kosmetischen Behandlung der Haut einschließlich der Kopfhaut, der Haare und/oder der Lippen, **dadurch gekennzeichnet, daß** auf die Haut, die Haare und/oder die Lippen eine Zusammensetzung nach einem der Ansprüche 1 bis 15 aufgetragen wird.
